# EUROPEAN PATENT SPECIFICATION

(11) **EP 0 291 502 B1**
(45) Date of publication and mention of the grant of the patent: **11.05.1994**
(21) Application number: 86905299.3
(22) Date of filing: 29.08.1986
(51) Int. Cl.: H05G 1/30

(54) **A METHOD AND DEVICE FOR CONTROLLING THE X-RADIATION OF AN X-RAY APPARATUS, IN PARTICULAR THAT OF A MAMMOGRAPHIC APPARATUS**
VERFAHREN UND VORRICHTUNG ZUR STEUERUNG DER RÖNTGENSTRAHLUNG EINER RÖNTGENSTRAHLVORRICHTUNG, INSBESONDERE EINES MAMMOGRAPHEN
PROCEDE ET DISPOSITIF POUR REGULER LE RAYONNEMENT-X D'UN APPAREIL A RAYONS-X, NOTAMMENT CELUI D'UN APPAREIL DE MAMMOGRAPHIE

(30) Priority: 29.08.1985 FI 853317
(43) Date of publication of application: 23.11.1988
(73) Proprietor: Dentsply Research & Development Corp., Milford Delaware 19963 (US)
(72) Inventor: JÄRVINEN, Erkki, SF-00530 Helsinki (FI); AARNIO, Jaakko, SF-00910 Helsinki (FI); KARJALAINEN, Alpo, SF-00810 Helsinki (FI); WEDERHORN, Markku, SF-02130 Espoo (FI)
(74) Representative: Gordon, Richard John Albert
(86) International application number: FI8600092
(87) International publication number: WO8701555

(56) References cited:
- EP-A- 0 200 272
- SE-B- 420 459
- US-A- 2 747 104
- US-A- 3 857 039
- US-A- 3 974 385
- US-A- 4 189 645
- US-A- 4 486 896
- DERWENT'S ABSTRACT No. 84-181289/29, SU 1053334-A. (CHARON ET AL) 7 November 1983".

## Description

The invention relates to a method and device for controlling the X-radiation of an X-ray apparatus, in particular a mammographic apparatus. The purpose of the control is an automatic optimization of the ratio of the X-ray dose received by the patient to the information value of the x-radiogram.

When X-raying tissue, a certain portion of the radiation spectrum constitutes the main part of the information contained in the photograph. Too soft a radiation is to a major part absorbed into the tissue, thus unnecessarily increasing the patient's radiation dose. On the other hand, too hard a radiation passes through the tissue nearly without being absorbed, whereby no information on the tissue is formed in the photograph, but the radiation only weakens the contrast of the photograph, i.e. the information value.

In mammography, it is known (cf. e.g. the patent SE 420 459) to adjust the exposure according to the object to be measured, i.e. the compression thickness of the breast. However, e.g. the texture of the tissue or corresponding circumstances influencing the penetration of the rays, have not then been considered. Thus it has often been necessary to repeat the X-photography with other values, whereby the radiation received by the patient has grown unnecessarily.

The purpose of the invention is to achieve a method and a device for mammography, in which the correct radiation spectrum is secured according to the respective tissue quality.

The invention is based on the observation that considering the entire X-raying sequence, even a very short sample of the penetrated radiation provides sufficient information on the quality of the tissue, and thus the spectrum may be correctly adjusted on the basis of the sampling carried out during the first moments of the X-ray photography. It may be noted in this context that it is prior known through panoramic X-ray devices (cf e.g. patent specification JP-142 045/1983) to continuously adjust the voltage of an X-ray tube on the basis of the penetrated radiation, however in this case, the purpose of the control is to create a homogeneous degree of blackening throughout the entire panoramic area.

EP-A-0 200 272 discloses a method for adjusting the X-radiation of an X-ray device wherein the object is positioned correctly for photographing; certain initial parameters (predetermined exposure density, exposure duration) for the X-ray device are chosen before photographing; a sample is taken prior to the X-photography of the radiation having penetrated the object, whereby an adjustment of the tube current is made; and on the basis of this sample and the tube current used for this sample, the radiation spectrum is adjusted for the X-ray photography. EP-A-0 200 272 has a priority date of 29 April 1985 and although it was not published until 5 November 1988 it is admissible under Article 54(3) EPC.

The method of the present invention is characterised in that, at the very beginning of the X-ray photography, a very short sample with regard to the entire X-raying sequence is taken of the radiation having penetrated the object, and on the basis of this the radiation spectrum is adjusted for the period of the main sequence of the X-ray photography.

The short sampling may take place at the very beginning of the X-ray photography, as soon as the voltage of the tube has reached its initial set value, or then as a separate phase immediately preceding the X-raying phase itself. As the voltage of the X-ray tube rises to its final value e.g. in less than 10 ms, a few ms being enough for the sampling, it is understandable that this duration is of insignificant importance to the total exposure, which takes at least a few hundred ms. Performing the sampling as a separate phase becomes relevant especially when one wishes to correct the filtering as well, since it is not possible to change the filter during the X-ray photography without special filter constructions.

Another possibility of adjusting the spectrum is to change the point of focus on the anode, if the various areas of the anode plate have a different material composition.

The preferred embodiments of the method according to the invention are presented in the dependent claims 2-6.

A device according to the invention is correspondingly characterized in that a sensor detecting the radiation having penetrated the object of X-ray photography is provided, as well as a generative coupling starting from the sensor for adjusting the voltage of the X-ray tube or the filter, or for adjusting the focus point on the basis of radiation detected at the beginning of the radiation sequence in an anode of a non-homogenous material.

The preferred embodiments of the device according to the invention are presented in claims 8-10.

Advantageously the X-ray photography is controlled by means of microprocessors, which regulate any of the above parameters on the basis of the penetration effect measured at the beginning of the radiation, as well as the time of exposure on the basis of the total radiation integrated with regard to the time.

The beneficial spectrum is a function of the thickness of the object to be X-ray photographed, i.e. as the thickness grows, the spectrum used to derive the information becomes harder. Correspondingly, the optimal spectrum is also a function of the composition of the object. When X-ray photographing a close texture, the spectrum has to be harder than e.g. when X-ray photographing a fat tissue. For instance in mammography, the term "equivalent thickness" may be used, by which is meant the combined effect of the compression thickness of the breast and the composition on the optimal spectrum.

The spectrum can be hardened by increasing the voltage of the X-ray tube, thus increasing the maximum energy of the radiation, and simultaneously changing the radiation filter so as to filter a low-energy radiation more efficiently. By doing the contrary, the spectrum gets correspondingly softer. It is advantageous to choose the starting voltage, as well as the filter to use, on the basis of the thickness of the object of X-ray photography measured with the aid of a thickness gauge.

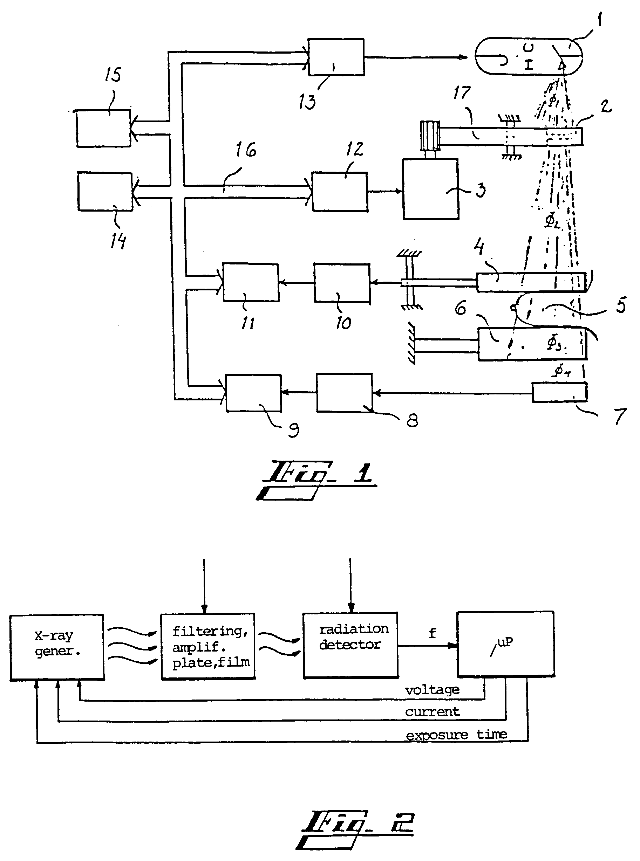

The invention is described in detail below with reference to the enclosed drawings, in which Figure 1 is a schematic perspective of the mammographic device according to the invention and Figure 2 is a block diagram of the adjusting method.

The following reference numerals are used in Figure 1:
1. X-ray tube
2. Filter disposed on the periphery of the disc
3. Motor rotating the disc (17)
4. Clamping sheet which provides the control compression thickness of the gauge
5. Breast to be X-rayed
6. Cassette casing containing the film to be exposed
7. Exposure automation sensor giving the signal to be amplified to the exposure automation unit
8. Exposure automation amplifier and voltage-to-frequency converter
9. Timer (Motorola 6840 or equiv) for the exposure automation (8)
10. Compression thickness gauge and voltage-to-frequency converter
11. Timer (Motorola 6840 or equiv.) for the compression thickness gauge
12. Control logic of the motor, determining the position of the motor and thus also that of the disc
13. Efficiency generator adjusting the voltage (U) and the current (I) of the X-ray tube 1
14. Microprocessor (Motorola 6803 or equiv.)
15. Program store (Intel 2764 or equiv.)
16. Address and data channel, along which the instructions and controls are transmitted
17. Filter disc.

For the adjustment, a compression thickness gauge 10 for the breast and a filter unit 17 are provided in the device, the filter unit comprising filters 2 which are of various materials and automatically replaceable by indexing rotation of the disc 17 constituting the filter unit. Additionally, the device comprises the exposure automation unit 7, 8, which measures the exposure rate of the radiation having penetrated the breast 5 in relative units.

Before the X-ray photography, the microprocessor unit 14 selects, on the basis of the compression thickness of the breast 5, the appropriate filter and a first approximation for the voltage of the X-ray tube 1.

The most reliable information about the texture is obtained during the X-ray photography from the response of the exposure automation 7, 8. From the voltage U and current I of the X-ray tube as well as the filtering of the radiation, the spectrum of the radiation attaining the breast and the exposure rate may be concluded. From the exposure automation response, i.e. the starting signal of the sensor 7, it is possible to derive the exposure rate of the radiation having penetrated the breast, and further the equivalent thickness of the breast. On the basis of this, a possible correction of the voltage U of the X-ray tube 1 may be carried out at the beginning of the X-ray photography, in order to optimize the X-ray photography result and the radiation dose of the patient.

The correction may be done e.g. in the following manner:
When the voltage of the X-ray tube has risen to its initial set value, for instance 5 - 10 ms after switching it on, a sample is taken from the starting signal of the sensor 7 for a short period of e.g. a few ms. As the output signal is converted into pulses by the converter 8, the sample comprises in practice a certain number of pulses. This is compared with the number programmed in the microprocessor, considered appropriate, and if necessary, a correction of the voltage is carried out. It is naturally even possible to carry out a correction of the filter during the X-ray photography, provided that the filter disc indexing rotation for filter exchange is fast enough. As stated above, the sampling can however in this case take place as a prephase of the X-ray photography, upon which the voltage is temporarily interrupted for the change of the filter. This does not change the device, but merely concerns the programming of the microprocessor.

An alternative embodiment of the above filter construction would be a unit consisting of superposed filters, in which each filter can be transferred into the radiation path e.g. by means of a controllable solenoid.

## Claims

1. A method for adjusting the intensity of X-radiation of an X-ray device comprising an X-ray tube (1), for example that of a mammographic device, wherein a subject to be X-rayed is positioned correctly for taking an X-ray photograph thereof during an X-ray sequence, and certain initial parameters for the X-ray device are chosen before photographing, characterized in that a sample whose duration is very short with regard to the duration of the entire X-raying sequence, of the radiation having penetrated the subject, is taken at the beginning of the X-raying sequence, and on the basis of this the radiation spectrum is adjusted for the remainder of the X-raying sequence.

2. A method according to claim 1, characterized in that said short duration sample is taken at the beginning of and as part of a continuous X-raying sequence, once the voltage of the X-ray tube of the X-ray device has risen to an initial set value, and in that the spectrum is adjusted on the basis of said sample by correcting the voltage of the tube.

3. A method according to claim 1, characterized in that said X-ray device includes a radiation filter (2); in that said short sample is taken in a separate prephase of the X-raying sequence, whereupon the X-raying sequence is interrupted; in that the radiation spectrum is adjusted by exchanging said radiation filter, and optionally by altering an initial set value of the voltage; and in that the X-raying sequence itself is then resumed.

4. A method according to claim 1, 2 or 3, characterized in that the X-ray tube (1) has an anode of a non-homogeneous material, and in that the radiation spectrum is adjusted by changing the focus point of the cathode ray in the anode.

5. A method according to any one of claims 1 to 4, characterized in that said sample of the radiation having penetrated the subject is taken by a radiation detector (7), which can be e.g. a semiconductor detector or an ionization chamber.

6. A method according to claim 5, characterized in that the output of the radiation detector (7) is converted into pulses by a voltage-to-frequency converter (8), by means of which, and of a regenerative circuit (9, 14, 15) the radiation spectrum is adjusted.

7. X-ray apparatus including a device for adjusting the X-radiation thereof, said X-ray apparatus having an X-ray tube (1), an X-ray tube generator (13) of adjustable output voltage connected to the X-ray tube, a radiation filter (2), and a photographing device (6) sensitive to X-rays, characterized in that it comprises a detector (7) for detecting X-radiation incident thereon, as well as a re-generative coupling (8, 9, 14, 15) responsive to the detector for adjusting the voltage of the X-ray tube (1) or selecting the appropriate filter (2), or for adjusting the point of focus on the anode of the X-ray tube where said anode is formed of a non-homogeneous material, on the basis of the X-radiation detected in the beginning of a radiation sequence being effected by the apparatus.

8. Apparatus according to claim 7, characterized in that it comprises additionally a thickness gauge (10) for measuring the thickness of an object being irradiated and photographed; and in that the output signal of said thickness gauge is used for selecting the initial value of the parameter to be adjusted.

9. Apparatus according to claim 7, characterized in that the apparatus includes a filtering unit comprising a rotatable circular disc (17), in which various radiation filters (2) are disposed; and in that the selection of the appropriate filter (2) is performed by rotatably indexing the disc with the aid of a motor (3) controlled by said regenerative coupling (8,9,14,15) by way of a control logic.

10. Apparatus according to claim 7, characterized in that the apparatus includes a filtering unit composed of superposed filters (2), of which each one is transferable separately or together with the others into the radiation path e.g. by means of an associated solenoid.

## Patentansprüche

1. Verfahren, um die Intensität von Röntgenstrahlung einer Röntgenvorrichtung einzustellen, die eine Röntgenröhre (1) aufweist, beispielsweise die einer Mammographievorrichtung, wobei ein zu röntgendes Objekt richtig positioniert wird, um während einer Röntgensequenz eine Röntgenaufnahme davon zu machen, und wobei bestimmte Anfangsparameter für die Röntgenvorrichtung vor der Aufnahme gewählt werden,
dadurch gekennzeichnet,
daß eine Probe der durch das Objekt hindurchgetretenen Strahlung, deren Dauer in bezug auf die Dauer der gesamten Röntgensequenz sehr kurz ist, zu Beginn der Röntgensequenz genommen wird,
und daß auf der Basis dieser Probe das Strahlungsspektrum für die restliche Röntgensequenz eingestellt wird.

2. Verfahren nach Anspruch 1,
dadurch gekennzeichnet,
daß die Probe kurzer Dauer zu Beginn und als Teil von einer kontinuierlichen Röntgensequenz genommen wird, sobald die Spannung der Röntgenröhre der Röntgenvorrichtung auf einen Anfangs-Vorgabewert gestiegen ist,
und daß das Spektrum auf der Basis der Probe eingestellt wird, indem man die Spannung der Röhre korrigiert.

3. Verfahren nach Anspruch 1,
dadurch gekennzeichnet,
daß die Röntgenvorrichtung ein Strahlungsfilter (2) aufweist;
daß die Probe kurzer Dauer in einer gesonderten Vorphase der Röntgensequenz genommen wird, woraufhin die Röntgensequenz unterbrochen wird;
daß das Strahlungsspektrum eingestellt wird, indem man das Strahlungsfilter austauscht und indem man wahlweise einen Anfangs-Vorgabewert der Spannung ändert;
und daß die Röntgensequenz selbst dann wieder aufgenommen wird.

4. Verfahren nach Anspruch 1, 2 oder 3,
dadurch gekennzeichnet,
daß die Röntgenröhre (1) eine Anode aus einem nichthomogenen Material hat
und daß das Strahlungsspektrum eingestellt wird, indem man den Brennpunkt des Kathodenstrahls auf der Anode ändert.

5. Verfahren nach einem der Ansprüche 1 bis 4,
dadurch gekennzeichnet,
daß die Probe der durch das Objekt hindurchgetretenen Strahlung von einem Strahlungsdetektor (7) aufgenommen wird, der beispielsweise ein Halbleiterdetektor oder eine Ionisationskammer sein kann.

6. Verfahren nach Anspruch 5,
dadurch gekennzeichnet,
daß das Ausgangssignal des Strahlungsdetektors (7) von einem Spannungs-Frequenz-Umsetzer (8) in Impulse umgesetzt wird, mit Hilfe dessen und eines Rückkopplungskreises (9, 14, 15) das Strahlungsspektrum eingestellt wird.

7. Röntgenvorrichtung, die eine Einrichtung aufweist, um ihre Röntgenstrahlung einzustellen, wobei die Röntgenvorrichtung folgendes aufweist:
eine Röntgenröhre (1),
einen Röntgenröhrengenerator (13) mit einstellbarer Ausgangsspannung, der mit der Röntgenröhre verbunden ist, einen Strahlungsfilter (2) und
eine für Röntgenstrahlen empfindliche Aufnahmeeinrichtung (6),
dadurch gekennzeichnet,
daß sie einen Detektor (7) zum Abtasten von darauf auftreffender Röntgenstrahlung sowie eine Rückkopplungseinrichtung (8, 9, 14, 15) aufweist, die auf den Detektor anspricht, um auf der Basis der Röntgenstrahlung, die zu Beginn einer von der Vorrichtung bewirkten Strahlungssequenz gemessen wird, die Spannung der Röntgenröhre (1) einzustellen oder das geeignete Filter (2) zu wählen, oder um den Brennpunkt auf der Anode der Röntgenröhre einzustellen, wenn die Anode aus einem nichthomogenen Material gebildet ist.

8. Vorrichtung nach Anspruch 7,
dadurch gekennzeichnet,
daß sie zusätzlich einen Dickenmesser (10) aufweist, um die Dicke eines bestrahlten und aufgenommenen Objektes zu messen;
und daß das Ausgangssignal des Dickenmessers verwendet wird, um den Anfangswert des einzustellenden Parameters zu wählen.

9. Vorrichtung nach Anspruch 7,
dadurch gekennzeichnet,
daß die Vorrichtung eine Filtereinheit aufweist, die eine drehbare kreisförmige Scheibe (17) aufweist, in der verschiedene Strahlungsfilter (2) angeordnet sind;
und daß die Wahl des geeigneten Filters (2) vorgenommen wird durch drehbares Weiterschalten der Scheibe mit Hilfe eines Motors (3), der von der Rückkopplungseinrichtung (8, 9, 14, 15) über eine Steuerlogik gesteuert wird.

10. Vorrichtung nach Anspruch 7,
dadurch gekennzeichnet,
daß die Vorrichtung eine Filtereinheit aufweist, die aus übereinander angeordneten Filtern (2) besteht, von denen jedes, beispielsweise durch ein zugeordnetes Solenoid, einzeln oder gemeinsam mit den anderen in den Strahlengang überführbar ist.

## Revendications

1. Procédé pour réguler l'intensité du rayonnement X d'un appareil à rayons X, comprenant un tube à rayons X (1), par exemple celui d'un appareil de mammographie, dans lequel un patient à radiographier est positionné correctement pour subir une radiographie pendant une phase d'irradiation, et dans lequel certains paramètres initiaux de l'appareil à rayons X sont déterminés avant la radiographie, caractérisé en ce qu'un échantillon du rayonnement ayant pénétré dans le patient, dont la durée est très courte par rapport à la durée totale de la phase d'irradiation, est prélevé au début de la phase d'irradiation, et en ce que ledit échantillon permet d'ajuster le spectre du rayonnement pour le reste de la phase d'irradiation.

2. Procédé selon la revendication 1, caractérisé en ce que ledit échantillon de courte durée est prélevé au début de et en tant que partie intégrante d'une phase d'irradiation continue, une fois que la tension du tube à rayons X du dispositif à rayons X a atteint une valeur de consigne initiale, et en ce que le spectre est ajusté en fonction dudit échantillon par correction de la tension du tube.

3. Procédé selon la revendication 1, caractérisé en ce que ledit dispositif à rayons X comprend un filtre de rayonnement (2) ; en ce que ledit échantillon de courte durée est prélevé lors d'une phase préliminaire indépendante de la phase d'irradiation, à la suite de quoi la phase d'irradiation est interrompue ; en ce que le spectre du rayonnement est ajusté par changement dudit filtre de rayonnement, et éventuellement par modification d'une valeur de consigne initiale de la tension ; et en ce que la phase d'irradiation à proprement parler reprend ensuite.

4. Procédé selon la revendication 1, 2 ou 3, caractérisé en ce que le tube à rayons X (1) est doté d'une anode en un matériau non-homogène, et en ce que le spectre du rayonnement est ajusté par changement du point focal du rayon cathodique dans l'anode.

5. Procédé selon l'une quelconque des revendications 1 à 4, caractérisé en ce que ledit échantillon de rayonnement ayant pénétré dans le patient est prélevé par un détecteur de rayonnement (7), qui peut, par exemple, être un détecteur à semi-conducteurs ou une chambre d'ionisation.

6. Procédé selon la revendication 5, caractérisé en ce que la sortie du détecteur de rayonnement (7) est convertie en impulsions par un convertisseur tension-fréquence (8), qui permet, conjointement à un montage à réaction (9, 14, 15), d'ajuster le spectre du rayonnement.

7. Appareil à rayons X incluant un dispositif pour réguler les rayons X dudit appareil, ledit appareil à rayons X comportant un tube à rayons X (1), un générateur de tube à rayons X (13) de tension de sortie ajustable, relié au tube à rayons X, un filtre de rayonnement (2), et un dispositif de photographie (6) sensible aux rayons X, caractérisé en ce qu'il comprend un détecteur (7) permettant de détecter les rayons X qui le frappent, ainsi qu'un montage à réaction (8, 9, 14, 15) commandé par le détecteur pour ajuster la tension du tube à rayons X (1) ou sélectionner le filtre approprié (2), ou pour régler le point focal de l'anode du tube à rayons X, lorsque ladite anode est formée d'un matériau non-homogène, sur la base du rayonnement X détecté au début d'une phase d'irradiation effectuée au moyen de l'appareil.

8. Appareil selon la revendication 7, caractérisé en ce qu'il comprend en outre une jauge d'épaisseur (10) permettant de mesurer l'épaisseur d'un objet en train d'être irradié et photographié ; et en ce que le signal de sortie de ladite jauge d'épaisseur est utilisé pour sélectionner la valeur initiale du paramètre à ajuster.

9. Appareil selon la revendication 7, caractérisé en ce que l'appareil comporte une unité de filtrage comprenant un disque circulaire rotatif (17), dans lequel sont disposés différents filtres de rayonnement (2) ; et en ce que la sélection du filtre approprié (2) est assurée par indexage rotatif du disque à l'aide d'un moteur (3) commandé par ledit montage à réaction (8, 9, 14, 15) au moyen d'une logique de commande.

10. Procédé selon la revendication 7, caractérisé en ce que l'appareil comporte une unité de filtrage composée de filtres superposés (2), dont chacun peut être transféré indépendamment ou conjointement avec les autres sur la trajectoire de rayonnement, par exemple au moyen d'un électro-aimant associé.
